(19) Europäisches Patentamt
European Patent Office
Office européen des brevets

(11) Veröffentlichungsnummer: **0 125 415**
**B1**

(12) # EUROPÄISCHE PATENTSCHRIFT

(45) Veröffentlichungstag der Patentschrift: **26.11.86**

(51) Int. Cl.⁴: **C 07 D 207/32**

(21) Anmeldenummer: **84102635.4**

(22) Anmeldetag: **10.03.84**

(54) **Verfahren zur Herstellung von Pyrrolen.**

(30) Priorität: **16.03.83 DE 3309355**

(43) Veröffentlichungstag der Anmeldung:
**21.11.84 Patentblatt 84/47**

(45) Bekanntmachung des Hinweises auf die Patenterteilung:
**26.11.86 Patentblatt 86/48**

(84) Benannte Vertragsstaaten:
**BE CH DE FR GB LI**

(56) Entgegenhaltungen:
**JOURNAL OF CHEMICAL SOCIETY CHEMICAL COMMUNICATIONS, Nr. 22, 1974, London, GB; S.-I. MURAHASHI et al. "Conversion of alcohols into unsymmetrical secondary or tertiary amines by a palladium catalyst. Synthesis of N-substituted pyrroles", Seiten 931-932**
**Chemical Abstracts Band 41, Nr. 17, 10. September 1947, Columbus, Ohio, USA; Spalte 5552b-d**

(73) Patentinhaber: **BASF Aktiengesellschaft, Carl-Bosch- Strasse 38, D-6700 Ludwigshafen (DE)**

(72) Erfinder: **Menig, Helmuth, Dr., Hauptstrasse 22, D-6701 Friedelsheim (DE)**
Erfinder: **Fischer, Martin, Dr., Elbingerweg 1, D-6700 Ludwigshafen 29 (DE)**
Erfinder: **Baer, Karl, Dr., Kastanienweg 1, D-6940 Weinheim (DE)**

EP 0 125 415 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von Pyrrolen durch Umsetzung von Ammoniak oder Aminen mit 2-Butendiolen-1,4 in Gegenwart von Trägerkatalysatoren mit Kupfer, Silber, Zink, Palladium, Nickel, Kobalt und/oder Platin und/oder Verbindungen dieser Metalle als Katalysatoren und gegebenenfalls in Gegenwart von Verbindungen des Chroms und/oder Mangans als Zusatzkatalysatoren in der Gasphase bei einer Temperatur von 180 bis 500°C.

Pyrrole werden technisch durch katalytische Dehydrierung, z.B. an $Pd/Al_2O_3$-Katalysatoren, der entsprechenden Pyrrolidine hergestellt. Diese wiederum sind z.B. aus Butandiol-1,4 bzw. Tetrahydrofuran und Ammoniak bzw. Alkylaminen zugänglich (Ullmanns Encycl. d. techn. Chem. (1980), Band 19, Seiten 639 bis 642). Nachteilig an diesem Verfahren ist im Hinblick auf einfachen und wirtschaftlichen Betrieb die Notwendigkeit einer Arbeitsweise in zwei Schritten, ausgehend von 1,4-Diolen bzw. Tetrahydrofuran, verbunen mit einer Isolierung des entsprechenden Pyrrolidins, z.B. durch Destillation.

Die US-PS 2 421 650 lehrt, daß sich Pyrrole durch Umsetzung von 2-Butin-1,4-diolen mit Ammoniak oder primären Aminen in Gegenwart von wasserabspaltenden Katalysatoren wie beispielsweise Aluminiumoxid in der Gasphase bei 200 bis 400°C herstellen lassen. Ausgehend von 2-Buten-1,4-diolen bzw. Butan-1,4-diolen werden hingegen nicht Pyrrole sondern Pyrroline bzw. Pyrrolidine erhalten, die in einem zweiten Schritt katalytisch dehydriert werden müßten, um zum gewünschten Endprodukt zu gelangen.

Eine einstufige Synthese von N-Alkylpyrrolen wird in J.C.S., Chem. Comm., 1974, 931 bis 932 beschrieben. Man setzt in flüssiger Phase 2-Buten-diol-1,4 mit Cyclohexylamin bzw. anderen höhersiedenden Aminen in Gegenwart von Palladium-Mohr während 14 bis 20 Stunden bei 120°C um:

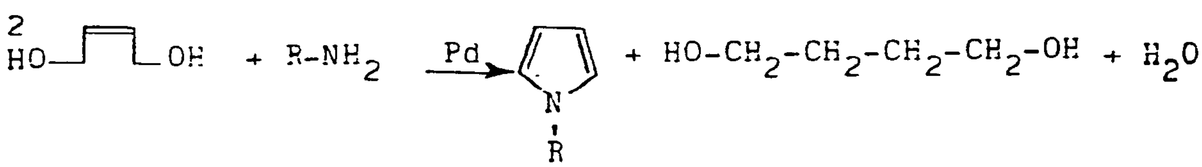


Das Verfahren besitzt jedoch folgende Nachteile: Palladium-Mohr ist in den eingesetzten Mengen von ca. 3 Gew.%, bezogen auf 2-Butandiol-1,4, ein teurer Katalysator. Pro Mol gebildetes Pyrrol fällt 1 Mol Butandiol-1,4 an. Die Reaktionsgeschwindigkeit ist sehr gering (Raum-Zeit-Ausbeute unter 15 g Pyrrol/liter Reaktionvolymen und Stunde).

Es wurde nun gefunden, daß man Pyrrole der Formel

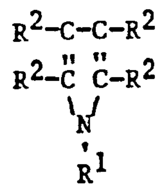
I,

worin die einzelnen Reste $R^1$ und $R^2$ gleich oder verschieden sein können und jeweils ein Wasserstoffatom, einen aliphatischen, cycloaliphatischen, araliphatischen oder aromatischen Rest bedeuten, durch Umsetzung von Stickstoffverbindungen uit 2-Butendiolen-1,4 in Gegenwart von Katalysatoren, vorteilhaft erhält, wenn man Ammoniak oder Amine der Formel

$$R^1-NH_2 \qquad II,$$

worin $R^1$ die vorgenannte Bedeutung besitzt, mit 2-Butendiolen-1,4 der Formel

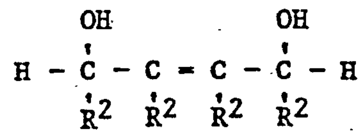

III,

worin $R^2$ die vorgenannte Bedeutung besitzt, in Gegenwart von
a) Kupfer, Silber, Zink, Palladium, Nickel, Kobalt und/oder Platin und/oder
b) Verbindungen dieser Metalle als Katalysatoren und
c) gegebenenfalls in Gegenwart von Verbindungen des Chroms und/oder Mangans als Zusatzkatalysatoren
d) auf einem Träger
in der Gasphase bei einer Temperatur von 180 bis 500° C umsetzt.

Die Umsetzung kann für den Fall der Verwendung von Methylamin und 2-Buten-diol-1,4 durch die folgenden Formeln wiedergegeben werden:

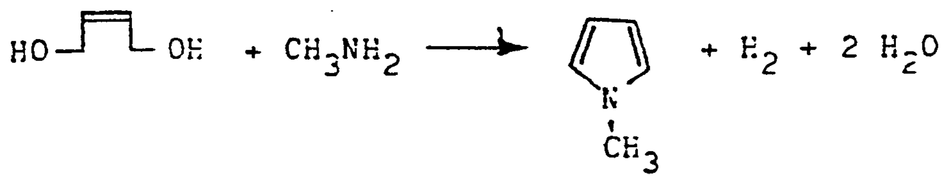

Im Hinblick auf den Stand der Technik liefert das Verfahren nach der Erfindung auf einfacherem und wirtschaftlicherem Wege Pyrrole in guter Ausbeute und Reinheit und in besserer Raum-Zeit-Ausbeute. Im allgemeinen werden erfindungsgemäß Raum-Zeit-Ausbeuten von 54 bis 165 g Pyrrol je Liter Trägerkatalysator und Stunde erhalten. Die Katalysatoren bzw. die zu verwendenden Katalysatormengen sind im Hinblick auf die bekannten Verfahren wirtschaftlicher. Das erfindungsgemäße Verfahren ist insbesondere auch im großtechnischen Maßstab und kontinuierlichen Betrieb geeignet. Alle diese Vorteile des erfindungsgemäßen Verfahrens sind im Hinblfck auf den Stand der Technik überraschend. Man hätte gerade im Vergleich mit dem im J.C.S. (loc. cit) beschriebenen Verfahren erwartet, daß bei den erfindungsgemäßen höheren Temperaturen schlechtere Ausbeuten erzielt und außerdem die Hälfte des Ausgangsstoffs z, Butandiolen-1,4 umgesetzt würden.

Die Ausgangsstoffe II werden mit den Ausgangsstoffen III in stöchiometrischer Menge oder im Überschuß, vorzugsweise in einer Menge von 0,1 bis 10 Mol, insbesondere 1 bis 4 Mol Ausgangsstoff II je Mol Ausgangsstoff III umgesetzt. Bevorzugte Ausgangsstoffe II und III und dementsprechend bevorzugte Endstoffe I sind solche, in deren Formeln die einzelnen Reste $R^1$ und $R^2$ gleich oder verschieden sein können und jeweils ein Wasserstoffatom, einen Alkylrest mit 1 bis 8 Kohlenstoffatomen, einen Cycloalkylrest mit 5 bis 8 Kohlenstoffatomen, einen Alkylarylrest oder einen Aralkylrest mit 7 bis 12 Kohlenstoffatomen oder einen Phenylrest bedeuten. Die vorgenannten Reste können noch durch unter den Reaktionsbedingungen inerte Gruppen, z.B. Alkylgruppen mit 1 bis 4 Kohlenstoffatomen, substituiert sein.

Neben Ammoniak eignen sich unzersetzt verdampfbare, primäre Amine zur Herstellung der entsprechenden Pyrrole I, beispielsweise Methylamin, Ethylamin, n-Propylamin, i-Propylamin, n-Butylamin, i-Butylamin, s-Butylamin, n-Pentylamin, 3-Methyl-1-butylamin, 3-Methyl-2-butylamin, 2-Methyl-2-butylamin, n-Hexylamin, n-Octylamin, 2-Ethyl-1-hexylamin, Cyclohexylamin, Benzylamin, Anilin, 2-Phenylethylamin, Toluidin.

Für die erfindungsgemäße Reaktion kommen beispielsweise folgende Ausgangsstoffe III in Betracht: 2-Butendiol-1,4, 2-Methyl-2-butendiol-1,4, 2,3-Dimethyl-2-butendiol-1,4, 2-Pentendiol-1,4, 2-Methyl-2-pentendiol-1,4, 2-Hexendiol-1,4, 3-Hexendiol-2,5, 2-Heptendiol-1,4, 3-Heptendiol-2,5, 2-Octendiol-1,4, 3-Octendiol-2,5, 4-Octendiol-3,6, 1-Phenyl-2-butendiol-1,4, 1-Phenyl-2-pentendiol-1,4, 1-Phenyl-3-pentendiol-2,5, 1-Phenyl-3-hexendiol-2,5, 1-Cyclohexyl-2-butendiol-1,4, 1-(p-Tolyl)-2-butendiol-1,4.

Als Beispiele für aus den Ausgangsstoffen II und III herstellbare Endstoffe I seien hier genannt: Pyrrol, 1-Methylpyrrol, 1-Ethylpyrrol, 1-n-Propylpyrrol, 1-i-Propylpyrrol, 1-n-Butylpyrrol, 1-i-Butylpyrrol, 1-s-Butylpyrrol, 1-n-Pentylpyrrol, 1-(3-Methyl-1-butyl)-pyrrol, 1-(3-Methyl-2-butyl)-pyrrol, 1-(2-Methyl-2-butyl)-pyrrol, 1-n-Hexylpyrrol, 1-n-Octylpyrrol, 1-(2-Ethyl-1-hexyl)-pyrrol, 1-Cyclohexylpyrrol, 1-Benzyl-pyrrol, 1-Phenylpyrrol, 1-(2-Phenylethyl)-pyrrol, 3-Methylpyrrol, 3,4-Di-methylpyrrol, 2-Methylpyrrol, 2,4-Dimethylpyrrol, 2,5-Dimethylpyrrol, 1,2-Dimethylpyrrol, 1,3-Dimethylpyrrol, 1,2,4-Trimethylpyrrol, 1,3,4-Trimethylpyrrol, 1,2,5-Trimethylpyrrol, 2-Ethylpyrrol, 1-Methyl-2-ethylpyrrol, 2-Phenylpyrrol, 1-Methyl-2-phenylpyrrol, 2-Benzylpyrrol, 1-Methyl-2-benzylpyrrol, 2-Cyclohexylpyrrol, 2-(p-Tolyl)-pyrrol.

Die Umsetzung wird bei einer Temperatur von 180 bis 500, vorzugsweise von 240 bis 350°C, drucklos oder unter Druck, kontinuierlich oder diskontinuierlich durchgeführt. Die Reaktionstemperatur soll so hoch sein, daß

das Reaktionsgemisch im gasförmigen Zustand gehalten wird. Dies kann bei höhersiedenden Diolen auch mit Hilfe eines Inertgases erzielt werden. Die Verweilzeiten betragen zweckmäßig von 1 bis vierzig Sekunden, bevorzugt von 5 bis 25 Sekunden. Zweckmäßig verwendet man keine zusätzliche, Lösungsmittel. Gegebenenfalls kann man das Amin II, z.B. Methylamin, in Gestalt seiner Lösung in einem geeigneten, unter den Reaktionsbedingungen inerten Lösungsmittel, z.B. Wasser, der Umsetzung zuführen. Zweckmäßig verwendet man in solchen Fällen das Lösungsmittel in einer Menge von 100 bis 10.000 Gew.% vorzugsweise von 100 bis 300 Gew.%, bezogen auf Ausgangsstoff II.

Die Ausgangsstoffe werden im allgemeinen verdampft und voneinander getrennt oder zweckmäßig als Gemisch im dampfförmigen Zustand der Reaktion zugeführt. Gegebenenfalls kann man als Verdünnungsmittel des Gasgemischs unter den Reaktionsbedingungen inerte Gase verwenden. Ebenfalls kann man zur Unterstützung der Verdampfung solche inerte Gase durch einen oder beide Ausgangsstoffe während der Verdampfung leiten. Als unter den Reaktionsbedingungen inerte Gase (Inertgas) werden zweckmäßig Alkane wie Methan, Ethan, Propan, 2,2-Dimethylpropan, Butan, Pentan, Isobutan; bevorzugt Stickstoff, Wasserdampf und/oder Kohlendioxid; und entsprechende Gemische verwendet. Es kommen zweckmäßig mindestens 0,1, vorzugsweise von 0,2 bis 100, insbesondere von 0,2 bis 10 Gramm Inertgas je Gramm Ausgangsstoff II oder III in Betracht. Bevorzugt ist eine Strömungsgeschwindigkeit des durch das Reaktionsgemisch geleiteten Inertgas von 0,1 bis 100, vorzugsweise 0,1 bis 5 Mol pro Stunde je Mol Ausgangsstoff II.

Als Katalysatoren kommen Silber, Zink, Palladium, Nickel, Kobalt, Platin, Kupfer, als Metalle und/oder Verbindungen dieser Metalle, einzeln oder im Gemisch miteinander, in Gestalt von Trägerkatalysatoren in Betracht. Der Katalysator kann ein oder mehrere Metalle, oder ein oder mehrere Metallverbindungen, oder gemeinsam ein Metall und eine oder mehrere Metallverbindungen; oder mehrere Metalle und ein oder mehrere Metallverbindungen, enthalten. Die Kupferverbindungen können solche des ein- oder zweiwertigen Kupfers sein; entsprechend können auch die anderen Metalle in verschiedenen Wertigkeiten in ihren Verbindungen vorliegen. Als Verbindungen kommen zweckmäßig die Acetate, Formiate, Hydroxide, Nitrate, Phosphate, Sulfate, Oxalate, Carbonate und bevorzugt Oxide der vorgenannten Metalle in Betracht. Vorteilhaft verwendet man im Katalysator die Metalle Silber, Palladium, Platin, Kupfer, Nickel und Kobalt als Metall, das Metall Zink als Metallverbindung. Zweckmäßig sind Katalysatoren mit 1 bis 3 Komponenten (Metalle und/oder Metallverbindungen). In einer bevorzugten Ausführungsform werden Metallverbindungen mit dem Trägermaterial vereint und dann die Verbindung (Verbindungen) zum Metall (zu Metallen) reduziert. Als Trägerkatalysator wird hier ein Katalysator (Einzelmetall oder Einzelverbindung oder mehrere Komponenten) auf einem Träger (mit 1 oder mehreren Trägermaterialien) verstanden. Im allgemeinen verwendet man den Katalysator (ohne Träger) in einer Menge von 0,01 bis 10, bevorzugt 0,05 bis 1 Gew.% Gesamtmetall, bezogen auf Ausgangsstoff II; Gesamtmetall wird als Gesamtsumme von freiem und in irgendeiner Form, z.B. Salzform, Additionsverbindung, Komplexverbindung, Legierung, gebundenem Metall bzw. freien und gebundenen Metallen im Katalysator definiert. Im kontinuierlichen Betrieb verwendet man zweckmäßig Durchsätze von 0,1 bis 100, vorzugsweise von 1 bis 50 g Ausgangsstoff III je Gramm Katalysator (ohne Träger) und Stunde.

Gegebenenfalls kann man noch Verbindungen, vorteilhaft Oxide, des Chroms und/oder Mangans als Zusatzkatalysatoren, zweckmäßig in einer Menge von 1 bis 500, insbesondere 10 bis 200 Gew.% Zusatzmetall je Gewichtsmenge Katalysatormetall (Hauptkatalysator) verwendet. Im Falle der Verwendung von Zusatzkatalysatoren gelten die vorgenannten Parameterbereiche und Erläuterungen bezüglich Katalysatoren für den Hauptkatalysator, unter Gesamtmetall wird dann Gesamtmetall des Hauptkatalysators verstanden. Wie beim Hauptkatalysator wird das Zusatzmetall als Gesamtsumme der Zusatzmetallkomponenten verstanden, gleichgültig in welcher Form die eine oder mehrere Metallverbindungen im Zusatzkatalysator vorliegen.

Der Katalysator, gegebenenfalls zusammen mit der Zusatzkatalysatorverbindung, wird zweckmäßig auf einem Träger, vorteilhaft in einer Menge von 0,2 bis 25 Gew.%, bezogen auf die Gewichtsmenge des Trägers, aufgebracht. Als Träger komoen in Frage Kieselsäureverbindungen wie Silikate, z.B. Natriumaluminiumsilikat, Calciumaluminiumsilikat, Bleicherden, Fullererde, Tone, Kaolin, Allophane, Zeolithe, Montmorillonit, Bimsstein, Floridaerde, Quarz, Asbest, Mullit, Bentonit; gefällte Kieselsäure, Kieselgel, Kieselgur; Titandioxid, Zirkondioxid, Zinndioxid, Magnesiumoxid, Magnesit, Aktivkohle; Erdalkalisulfate oder Erdalkaliphosphate, z.B. die Calcium- oder Bariumsalze; Metalloxide, die beim Calcinieren mit einer Borverbindung die entsprechenden Metallborate bilden, z.B. Calciumoxid; oder entsprechende Gemische vorgenannter Trägermaterialien. Es kann vorteilhaft sein, Natrium- und/oder Kaliumcarbonate, zweckmäßig in Gestalt ihrer 0,5 bis 20 gew.%igen wäßrigen Lösungen dem Träger beizugeben, zweckmäßig sind Mengen von 0,5 bis 10 Gew.% Metallcarbonat, bezogen auf die Gewichtsmenge Träger. Die Herstellung der Trägerkatalysatoren wird nach den üblichen Verfahren z.B. durch Auftragen der Metallverbindung und gegebenenfalls der Zusatzmetallverbindung auf den Träger, Trocknen und Calcinieren, beispielsweise zwischen 400 und 1200°C in reduzierender, oxidierender oder inerter Atmosphäre, durchgeführt. Der Träger kann auch in seiner gewünschten geometrischen Form mit einer Lösung der Metallverbindung und gegebenenfalls der Zusatzmetallverbindung getränkt und getrocknet werden. Ebenfalls kann man das Trägermaterial mit der Metallverbindung und gegebenenfalls der Zusatzmetallverbindung und Wasser verkneten, in die gewünschte Form bringen, trocknen und bei einer Temperatur von 400 bis 1200°C calcinieren.

Vorteilhafte Herstellmöglichkeiten für den Katalysator sind z.B.

1) Auf strang- oder pulverförmiges $SiO_2$ wird das Katalysatormetall zusammen mit einem Zusatz (Natrium-Kaliumcarbonat) durch Tränken mit den Lösungen des Metallnitrate und Zusatzcarbonate und anschließendes Eindampfen bis zur Trockne aufgebracht. Danach wird der Trägerkatalysator 1 bis 8 Stunden bei 500°C

getempert und im Wasserstoffstrom bei 200° C reduziert.

2) Auf strang- oder pulverförmiges $SiO_2$ wird zunächst eine Zusatzverbindung (Natrium-, Kaliumcarbonat) durch Tränken mit ihrer wäßrigen Lösung aufgebracht und bei 150°C getrocknet. Der derart vorbehandelte Träger wird nun mit einer wäßrigen Nitratlösung des Metalls getränkt und durch 2 bis 24-stündiges Erhitzen bei 200°C im Wasserstoffstrom reduziert. Wird zum Tränken Palladium-(II)-chloridlösung genommen, wird zweckmäßig mit alkalischer Formalinlösung oder 5 gew.%iger Hydrazinlösung reduziert.

3) Kieselsäure und ein basisches Oxid (z.B. MgO) werden intensiv miteinander in einem Kneter vermischt. Diese Mischung wird 6 Stunden lang auf 450°C erhitzt, anschließend mit einer Lösung der Metallnitrate getränkt und 2 bis 12 Stunden lang bei 300° C im Wasserstoffstrom reduziert.

Die Teilchengrößen der Trägerkatalysatoren beträgt vorzugsweise von 0,05 bis 7 Millimeter. Die Form kann beliebig, z.B. in Pillen-, Zylinder- oder Strangform, kugelförmig oder körnig, gewählt werden.

Zweckmäßig werden die Trägerkatalysatoren in Splitt-, Gries- oder Kugelform in der Wirbelschicht eingesetzt, wobei vorteilhaft Katalysatorteilchen mit Korngrößen von 0,005 bis 3,0 mm, insbesondere von 0,1 bis 1,0 mm, verwendet werden. Die Schichthöhe des Katalysatorbettes wird zweckmäßig so gewählt, daß sich Verweilzeiten der Ausgangsstoffe II in der Kontaktschicht von 1 bis 100, vorzugsweise von 5 bis 25 Sekunden ergeben. Bezüglich der Herstellung der Katalysatoren wird auf Houben-Weyl, Methoden der organischen Chemie, Band 4/2, Seiten 142 ff. und Ullmanns Encyclopädie der technischen Chemie, Band 9, Seiten 271 ff. verwiesen.

Die Reaktion kann wie folgt durchgeführt werden: Die gasförmigen Ausgangsstoffe II und III, gegebenenfalls im Gemisch mit Inertgasen werden bei der Reaktionstemperatur über den Trägerkatalysator in einem Festbett geleitet. Das aus dem Reaktor dampfförmig austretende Reaktionsgemisch wird gegebenenfalls dann in einem Zyklon entstaubt, in einer gekühlten Vorlage kondensiert, wo sich in der Regel 2 Phasen bilden, und durch fraktionierte Destillation der Phasen der Endstoff abgetrennt. Nicht umgesetzter Ausgangsstoff kann der Reaktion wieder zugeführt werden.

In einer zweckmäßigen Ausführungsform des Verfahrens wird der Ausgangsstoff in einer Wirbelschicht bei der Reaktionstemperatur umgesetzt. Der Trägerkatalysator kann zweckmäßig durch Inertgas, einem Gemisch von dampfförmigem Ausgangsstoff III und/oder Ausgangsstoff II und Inertgas oder den Ausgangsstoffe, allein als Wirbelschichtgas bei vorzugsweise vermindertem Druck in einer Wirbelschicht gehalten werden. Entsprechend kann die Gesamtmenge oder eine Teilmenge der Ausgangsstoffe getrennt von dem Wirbelschichtgas in den Wirbelschichtreaktor eingeleitet werden. Man kann das Verfahren nach der Erfindung in einem einfachen oder unterteilten, offenen oder geschlossenen Wirbelschichtsystem mit und ohne Fließstaubzirkulation durchführen. Bezüglich Reaktoren, Durchführung, Verfahrensvarianten und Reaktionsbedingungen des Wirbelschichtverfahrens wird auf Ullmanns Encyclopädie der technischen Chemie, Band 1, Seiten 916 ff. verwiesen. Die Aufarbeitung des Reaktionsgemischs erfolgt in vorgenannter Weise.

Die nach dem Verfahren der Erfindung herstellbaren Pyrrole sind wertvolle Ausgangsstoffe für die Herstellung von Farbstoffen, Korrosionsinhibitoren, Pharmazeutica und Schädlingsbekämpfungsmitteln. Bezüglich der Verwendung wird auf vorgenannte Veröffentlichungen, auf US-PS 3 008 965 Ullmanns Encycl. d. techn. Chem. (1963), Band 14, Seiten 505 bis 510 und FR-PS 1 574 570 verwiesen.

Die Gewichtsprozentangaben der Katalysatorbestandteile in den Beispielen beziehen sich auf die Gewichtsmenge Trägerkatalysator (= Katalysator und Trägermaterial).

**Beispiel 1**

Die Umsetzung erfolgte in einem senkrecht stehenden, elektrisch beheizten Rohrreaktor mit einem inneren Durchmesser von 28 mm und einer Höhe von 450 mm. In das Reaktionsrohr wird ein Trägerkatalysator (198 Gramm, 350 ml), der 8 Gew.% metallisches Kupfer auf Bims (Bims-Steinchen mit 3 bis 5 mm Durchmesser) enthält, eingefüllt und auf 250°C erhitzt. Über diesen Katalysator wurde kontinuierlich ein Gasgemisch aus 2-Buten-diol-1,4 und Monomethylamin im Gleichstrom geführt.

Die Einspeisegeschwindigkeiten betrugen 0,6 Mol Diol III pro Stunde und 0,66 Mol Amin II pro Stunde. Das Reaktionsprodukt wurde nach Verlassen des Reaktors auf 22°C abgekühlt. Es bildeten sich 2 Phasen. Die organische Phase enthielt nach gaschromatographischer Analyse 59 Gew.% 1-Methylpyrrol, die wäßrige Phase 3 Gew.% 1-Methylpyrrol, was einer Gesamtausbeute von 57 % entspricht. Die Abtrennung des Endstoffs erfolgte durch Destillation. Die Ergebnisse sind in Tabelle 1 aufgeführt.

**Beispiel 2**

Man verfuhr entsprechend Beispiel 1, verwendete aber einen Katalysator (248 Gramm; 410 ml) in Form von Strängen (4 mm Durchmesser, 10 mm Länge), der 0,5 Gew.% metallisches Palladium und 5 Gew.% metallisches Kupfer auf $SiO_2$ enthielt. Die Ergebnisse sind in Tabelle 1 aufgeführt.

**Beispiel 3**

Man verfuhr entsprechend Beispiel 1, verwendete aber einen Katalysator (192 Gramm; 325 ml), der 0,5 Gew.% metallisches Palladium und 5 Gew.% metallisches Silber auf $SiO_2$ enthielt. Die erhaltenen Ergebnisse sind in Tabelle 1 zusammengestellt.

**Beispiel 4**

Man verfuhr entsprechend Beispiel 1, verwendete aber einen Katalysator (223 Gramm; 350 ml), der 0,5 Gew.% metallisches Palladium, 5 Gew.% metallisches Silber und 5 Gew.% Magnesiumoxid auf $SiO_2$ enthielt. Die erzielten Ergebnisse sind in Tabelle 1 zusammengestellt.

**Beispiel 5**

Man verfuhr entsprechend Beispiel 1, verwendete aber einen Katalysator (207 Gramm; 350 ml), der 0,5 Gew.% metallisches Palladium, 5 % metallisches Silber und 2 Gew.% Kaliumoxid auf $SiO_2$ enthielt. Die erzielten Ergebnisse sind in Tabelle 1 zusammengestellt.

**Beispiel 6**

Man verfuhr entsprechend Beispiel 1, verwendete aber einen Katalysator (220 Gramm, 350 ml), der 0,5 Gew.% metallisches Palladium, 5 Gew.% metallisches Silber und 1,35 Gew.% $Mn_3O_4$ auf $SiO_2$ enthielt. Die erzielten Ergebnisse sind in Tabelle 1 zusammengestellt.

**Beispiel 7**

Man verfuhr entsprechend Beispiel 1, verwendete aber einen Katalysator (195 Gramm; 350 ml), der 5 Gew.% metallisches Silber auf $SiO_2$ enthielt. Die erzielten Ergebnisse sind in Tabelle 1 zusammengestellt.

**Beispiel 8**

Man verfuhr entsprechend Beispiel 1, verwendete aber einen Katalysator (181 Gramm; 410 ml), der 0,7 Gew.% metallisches Palladium auf $SiO_2$ enthielt. Die erzielten Ergebnisse sind in Tabelle 1 zusammengestellt.

**Beispiel 9**

Man verfuhr entsprechend Beispiel 1, verwendete aber einen Katalysator (825 Gramm; 170 ml), der 0,7 Gew.% metallisches Palladium und 2 Gew.% Natriumoxid auf $SiO_2$ enthielt. Die Einspeisegeschwindigkeit von 2-Buten-diol-1,4 betrug 0,45 Mol/h, die von wäßriger Methylamin-LÖsung (40 Gew.%), 0,68 Mol Methylamin/h. Die Ergebnisse sind in Tabelle 1 zusammengestellt.

**Beispiel 10**

Man verfuhr entsprechend Beispiel 1, verwendete aber einen Katalysator (358 Gramm; 405 ml), der 25,6 Gew.% metallisches Cobalt auf $SiO_2$ enthielt. Die erzielten Ergebnisse sind in Tabelle 1 zusammengestellt.

**Beispiel 11**

Man verfuhr entsprechend Beispiel 7, erhöhte aber die Einspeisegeschwindigkeit auf 0,9 Mol/h Diol III und 0,99 Mol/h Amin II. Die organische Phase des Reaktionsaustrags enthielt 90,1 Gew.%, die wäßrige Phase 3,0 Gew.% 1-Methylpyrrol, was einer Gesamtausbeute von 62 % entsprach.

**Beispiel 12**

Man verfuhr entsprechend Beispiel 7, verdoppelte aber die Einspeisegeschwindigkeit. Die erzielten Ergebnisse sind in Tabelle 1 zusammengestellt.

Tabelle 1

| Beispiel Nr. | Umsatz [%] | Ausbeute (% der Theorie) | Raum-Zeit-Ausbeute (g Pyrrol/l Katalysator und Stunde) |
|---|---|---|---|
| 1 | 99 | 57 | 78 |
| 2 | ca. 100 | 47 | 56 |
| 3 | 96 | 60 | 91 |
| 4 | 99 | 59 | 82 |
| 5 | 95 | 57 | 80 |
| 6 | 94 | 43 | 65 |
| 7 | 95 | 70 | 98 |
| 8 | 95 | 46 | 54 |
| 9 | 90 | 40 | 87 |
| 10 | 85 | 45 | 55 |
| 11 | 79 | 62 | 129 |
| 12 | 77 | 59 | 165 |

Der Endstoff der Beispiele 1 bis 13, 1-Methylpyrrol, hatte einen Kp. 110 bis 113° C.

**Beispiel 13**

Man verfuhr entsprechend Beispiel 1 unter Verwendung des in Beispiel 3 beschriebenen Katalysators (192 Gramm). Über diesen Katalysator führte man ein Gasgemisch aus 2-Methyl-2-butendiol-1,4 (0,51 Mol/h) sowie Methylamin (0,56 Mol/h). Die erzielten Ergebnisse sind in Tabelle 2 zusammengestellt.

**Beispiel 14**

Man verfuhr entsprechend Beispiel 1 unter Verwendung des in Beispiel 3 beschriebenen Katalysators (192 Gramm). Über diesen Katalysator leitete man ein Gasgemisch aus 2-Butendiol-1,4 (0,6 Mol/h) und n-Butylamin (0,6 Mol/h). Die erzielten Ergebnisse sind in Tabelle 2 zusammengestellt.

**Beispiel 15**

Man verfuhr entsprechend Beispiel 1 unter Verwendung des in Beispiel 3 beschriebenen Katalysators (192 Gramm). Über diesen Katalysator leitete man ein Gasgemisch aus 2-Butendiol-1,4 (0,6 Mol/h) und Cyclohexylamin-(0,6 Mol/h). Die erzielten Ergebnisse sind in Tabelle 2 zusammengestellt.

**Beispiel 16**

Man verfuhr entsprechend Beispiel 1 unter Verwendung des in Beispiel 3 beschriebenen Katalysators (192 Gramm). Über diesen Katalysator leitete man ein Gasgemisch aus 2-Butendiol-1,4 (0,61 Mol/h) und Ammoniak (0,66 Mol/h). Die erzielten Ergebnisse sind in Tabelle 2 zusammengestellt.

Tabelle 2

| Beispiel Nr. | Diol | Amin | Endstoff | Kp in °C (mbar) | Umsatz (%) | Ausbeute (% d.Th.) |
|---|---|---|---|---|---|---|
| 13 | 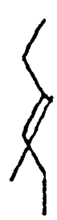 | $CH_3NH_2$ | 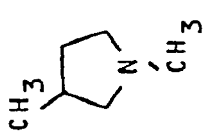 | 62 (87) | 85 | 59 |
| 14 | 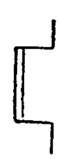 | $C_4H_9NH_2$ | 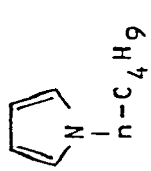 | 54 (10) | 86 | 53 |
| 15 | 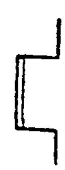 | 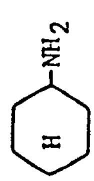 | 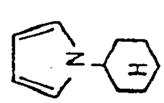 | 96-100 (11-13) | 88 | 41 |
| 16 | 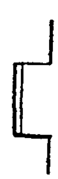 | $NH_3$ | 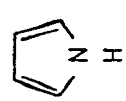 | 135 | 50 | 35 |

**Patentansprüche**

Verfahren zur Herstellung von Pyrrolen der Formel

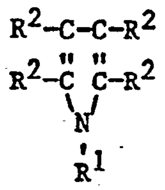 I,

worin die einzelnen Reste $R^1$ und $R^2$ gleich oder verschieden sein können und jeweils ein Wasserstoffatom, einen aliphatischen, cycloaliphatischen, araliphatischen oder aromatischen Rest bedeuten, durch Umsetzung von Stickstoffverbindungen mit 2-Butendiolen-1,4 in Gegenwart von Katalysatoren, dadurch gekennzeichnet, daß man Ammoniak oder Amine der Formel

$$R^1\text{-}NH_2 \qquad \text{II,}$$

worin $R^1$ die vorgenannte Bedeutung besitzt, mit 2-Butendiolen-1,4 der Formel

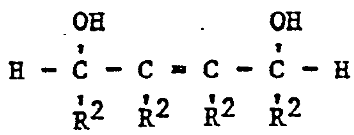 III,

worin $R^2$ die vorgenannte Bedeutung besitzt, in Gegenwart von
a) Kupfer, Silber, Zink, Palladium, Nickel, Kobalt und/oder Platin und/oder
b) Verbindungen dieser Metalle als Katalysatoren und
c) gegebenenfalls in Gegenwart von Verbindungen des Chroms und/oder Mangans als Zusatzkatalysatoren
d) auf einem Träger
in der Gasphase bei einer Temperatur von 180 bis 500°C umsetzt.

**Claim**

A process for the preparation of a pyrrole of the formula

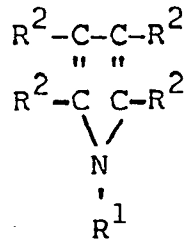

I,

where the individual radicals $R^1$ and $R^2$ can be identical or different and are each hydrogen or an aliphatic, cycloaliphatic, araliphatic or aromatic radical, by reacting a nitrogen compound with a but-2-ene-1,4-diol in the presence of a catalyst, wherein ammonia or an amine of the formula

$$R^1-NH_2 \quad II,$$

where $R^1$ has the above meanings, is reacted with a but-2-ene-1,4-diol of the formula

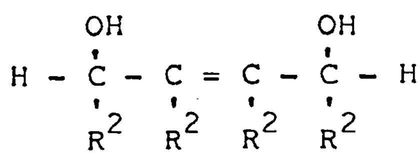

III,

where $R^2$ has the above meanings, in the presence of
a) copper, silver, zinc, palladium, nickel, cobalt and/or platinum and/or
b) compounds of these metals as catalysts, and in the presence or absence of
c) compounds of chromium and/or manganese as additional catalysts
d) on a carrier,
in the gas phase, at from 180 to 500°C.

**Revendication**

Procédé de préparation de pyrroles de formule

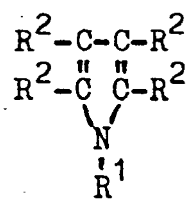

I

dans laquelle les différents radicaux $R^1$ et $R^2$ peuvent être identiques ou dissemblables et représentent chacun un atome d'hydrogène, un radical aliphatique, cycloaliphatique, araliphatique ou aromatique, par réaction de composés azotés avec des 2-butènediols-1,4 en présence de catalyseurs, caractérisé en ce que l'on fait réagir l'ammoniac ou des amines de formule

$$R^1\text{-}NH_2 \qquad\qquad II$$

dans laquelle $R^1$ a la signification donnée ci-dessus, avec des 2-butènediols-1,4 de formule

$$\begin{array}{ccccccccc} & & OH & & & & & & OH & & \\ & & | & & & & & & | & & \\ H & - & C & - & C & = & C & - & C & - & H \\ & & | & & | & & | & & | & & \\ & & R^2 & & R^2 & & R^2 & & R^2 & & \end{array} \qquad III$$

dans laquelle $R^2$ a la signification donnée ci-dessus, en présence

a) de cuivre, d'argent, de zinc, de palladium, de nickel, de cobalt et/ou de platine et/ou

b) de composés de ces métaux, servant de catalyseurs et

c) le cas échéant en présence de composés du chrome et/ou du manganèse servant de catalyseurs additionnels

d) sur un support, en phase gazeuse, à une température de 180 à 500° C.